# EUROPÄISCHE PATENTSCHRIFT

(11) **EP 1 420 789 B1**
(45) Veröffentlichungstag und Bekanntmachung des Hinweises auf die Patenterteilung: **25.04.2007**
(21) Anmeldenummer: 02767251.8
(22) Anmeldetag: 23.07.2002
(51) Int. Cl.: A61K 31/485, A61K 31/445, A61K 31/135

(54) **VERWENDUNG VON WIRKSTOFFEN MIT µ-OPIOID-REZEPTOR AGONISTISCHER WIRKUNG UND OPIOID-REZEPTOR ANTAGONISTISCHER WIRKUNG ALS KOMBINATIONSARZNEIMITTEL ZUR KREBSBEHANDLUNG**
USE OF ACTIVE INGREDIENTS HAVING A µ-OPIOID RECEPTOR AGONIST ACTION AND AN OPIOID RECEPTOR ANTAGONIST ACTION, AS COMBINATION DRUGS FOR THE TREATMENT OF CANCER
UTILISATION DE PRINCIPES ACTIFS A EFFET AGONISTE DU µ-RECEPTEUR OPIOIDE ET A EFFET ANTAGONISTE DU RECEPTEUR OPIOIDE, COMME MEDICAMENTS COMBINES DANS LE TRAITEMENT DU CANCER

(30) Priorität: 01.09.2001 DE 10142996
(43) Veröffentlichungstag der Anmeldung: 26.05.2004
(73) Patentinhaber: PAZ Arzneimittel- Entwicklungsgesellschaft mbH, 65933 Frankfurt am Main (DE)
(72) Erfinder: GEISSLINGER, Gerd, 65812 Bad Soden (DE); TEGEDER, Irmgard, Cambridge, MA 02139 (US)
(74) Vertreter: Wagner, Jutta
(86) Internationale Anmeldenummer: PCT/EP2002/008181
(87) Internationale Veröffentlichungsnummer: WO 2003/020277

(56) Entgegenhaltungen:
- US-A- 5 266 574
- US-B1- 6 277 384

## Beschreibung

Arzneimittel zur Behandlung von Krebserkrankungen wie Brustkrebs bei Mensch und Tier, welche entweder in einer Arzneiform oder in getrennten Arzneiformen Opioid-Rezeptoren-agonistische Wirkstoffe sowie Opioid-Rezeptoren-antagonistische Wirkstoffe enthalten und gleichzeitig oder zeitversetzt in geeigneter Dosis zur patientenoptimierten Therapie appliziert werden.

Krebserkrankungen sind in den Industrieländern eine der häufigsten Todesursachen. Die Häufigkeit der Erkrankung (Inzidenz) hängt von der Disposition (Veranlagung), der Exposition (Ausgesetztsein gegenüber krebserzeugenden Einflüssen wie Chemikalien, Strahlung, Viren, Stress, etc.) und dem Alter ab. Die Zunahme der Inzidenz für Krebserkrankungen im letzten Jahrhundert wird hauptsächlich der Zunahme der Lebenserwartung zugeschrieben. Wegen der Häufigkeit und Schwere der Krankheit wurden und werden intensive Forschungsarbeiten zur Vermeidung, Diagnose und Therapie von Krebs durchgeführt. Trotz dieser Bemühungen und den Maßnahmen zur Früherkennung haben sich, von Ausnahmen abgesehen, bei den am häufigsten auftretenden Krebsformen die Heilungschancen nur geringfügig verbessert.

Alle therapeutischen Maßnahmen beruhen auf einer Beseitigung oder einer Wachstumshemmung der entarteten Krebszellen. Klinische Möglichkeiten zur Rückbildung einer Tumorzelle in eine normale Gewebezelle stehen noch nicht zur Verfügung. Als medizinische Behandlungsverfahren kommen in Abhängigkeit von der Art der Krebserkrankung, dem Krankheitsstadium und dem Zustand des Patienten die Operation, die Bestrahlung und die Gabe von Medikamenten in Frage. Bei der medikamentösen Therapie werden zur Zerstörung oder Wachstumshemmung der Krebszellen Mitosehemmstoffe, alkylierende Zytostatika, zytostatisch wirksame Antibiotika, Antimetabolite, Hormone und Hormonantagonisten, Immunmodulatoren, Enzyme oder Radioisotope eingesetzt. Methoden zur gewebespezifischen medikamentösen Krebstherapie befinden sich ebenso im Entwicklungstadium wie gentherapeutische Maßnahmen. Wege zum selektiven Eingriff in die Signaltransduktion innerhalb des Zellzyklus der Krebszelle oder in die Angiogenese (Gefäßneubildung) innerhalb des Tumors befinden sich noch im Forschungsstadium.

Alle bisher bekannten medikamentösen Krebstherapien beruhen auf einem relativ unspezifischen Eingriff in den Zellzyklus der Krebszelle. Stets sind auch die gesunden Zellen vom Angriff der Medikamente mit betroffen. Deshalb treten in Abhängigkeit vom Wirkungsmechanismus der Substanz unterschiedliche, meistens jedoch erhebliche unerwünschte Arzneimittelwirkungen auf.

Aufgabe der Erfindung war es, Wirkstoffe zur Krebsbehandlung zu finden, die bereits als Arzneimittel für andere Anwendungsgebiete bekannt sind. Diese Wirkstoffe sollten ähnliche erwünschte Wirkungen auf die Tumorzelle ausüben wie die bisher eingesetzten Krebstherapeutika und dabei nach Möglichkeit ein günstigeres Nebenwirkungsspektrum aufweisen.

Wegen der intensiven internationalen Forschungsbemühungen, insbesondere des NCI (National Cancer Institute, USA), aus sämtlichen verfügbaren chemischen und natürlichen Stoffen potentielle Krebsmittel herauszufinden, schienen die Chancen zur Identifizierung von Krebsmitteln aus den bekannten Arzneistoffen sehr niedrig zu sein. Überraschenderweise gelang dies nun jedoch bei der gleichzeitigen Anwendung des bekannten starken Schmerzmittels (Opioids) Morphin und des Opioid-Antagonisten Naloxon. Durch anschließende systematische Untersuchungen konnte dieses Therapieprinzip auf andere, vom Wirkungsmechanismus her ähnliche, Wirkstoffe, d.h. Opioide und Opioidantagonisten, übertragen werden.

In der wissenschaftlichen Literatur finden sich aus Rezeptorbindungsstudien, Zellkulturversuchen oder Tierversuchen einige Hinweise darüber, dass Morphin bzw. ähnliche Substanzen in die Regulation des Zellüberlebens von Nichttumorzellen, aber auch die Tumorzellbiologie eingreifen können und Opiatantagonisten, wie Naloxon oder Naltrexon, diese Wirkung aufheben [Polakiewicz, R.D., Schieferl, S.M., Gingras, A.C., Sonenberg, N. & Comb, M.J. "mu-Opiod receptor activates signaling pathways implicated in cell survival and translational control" J Biol Chem 273, 23534 - 41 (1998); Maneckjee, R & Minna, J.D. "Opioids induce while nicotine suppresses apoptosis in human lung cancer cells" Cell Growth Differ 5, 1033 - 40 (1994); Maneckjee, R. & Minna, J.D. "Nonconventional opioid binding sites mediate growth inhibitory effects of methadone on human lung cancer cells" Proc Natl Acad Sci USA 89, 1169 - 73 (1992); Zagon, I.S. & McLaughlin, P.J. "Heroin prolongs survival time and retards tumor growth in mice with neuroblastoma" Brain Res Bull 7, 25 - 32 (1981); Sueoka, N., Sueoka, E., Okabe, S. & Fujiki, H. "Anti-cancer effects of morphine through inhibition of tumor necrosis factor-alpha release and mRNA expression" Carcinogenesis 17, 2337 - 41 (1996)]. US 6,277,384 beschreibt oral zu applizierende Kombinationen aus Opioid-Agonisten und Opioid-Antagonisten zur Schmerzbehandlung. Diese Kombinationen sollen den Missbrauch durch opioidabhängige Personen dadurch verhindern, dass sie bei diesem Personenkreis Abstinenzsyndrome verursachen, die der Anwender bei der missbräuchliche Opioideinnahme gerade verhindern möchte. US 5,266,574 beschreibt die Wachstumsbeschleunigung von pflanzlichen, tierischen oder menschlichen Zellen durch Behandlung mit Opioid Antagonsiten. Der immer noch sehr restriktive Einsatz von Morphin oder der verwandten Substanzen in der Schmerzbehandlung von Krebspatienten ließ es jedoch nicht als gerechtfertigt erscheinen, diese Substanzen zur Krebsbehandlung einzusetzen, wenn die Patienten nicht gleichzeitig dieser Schmerztherapie bedurften. Insbesondere das Abhängigkeitspotential und eine hemmende Wirkung auf das Atemzentrum sprechen dagegen, Morphin oder ähnliche Stoffe bei Patienten einzusetzen, die schmerzfrei sind oder mit anderen Schmerzmitteln ausreichend therapiert werden können, insbesondere wenn für die Tumorbehandlung höhere und daher toxischere Dosierungen erforderlich sind. Aus dieser Literatur läßt sich weiterhin entnehmen, daß Naloxon oder andere, die Giftigkeit von Opioiden vermindernden Opioid-Antagonisten, auch die tumorwachstumsinhibierende Wirkung der Opioide bei den untersuchten Lungentumor-Zellkulturen aufheben. Dieser Weg die Opioidmenge zu erhöhen schien daher nicht gangbar. Überraschenderweise konnte jedoch am Ganztier festgestellt werden, daß dies anders reagiert, als aus den Zellkulturversuchen zu vermuten war.

Die Erfindung betrifft die basiert auf Verwendung gemäß Anspruch 1 und den nachfolgend beschriebenen Ergebnissen.

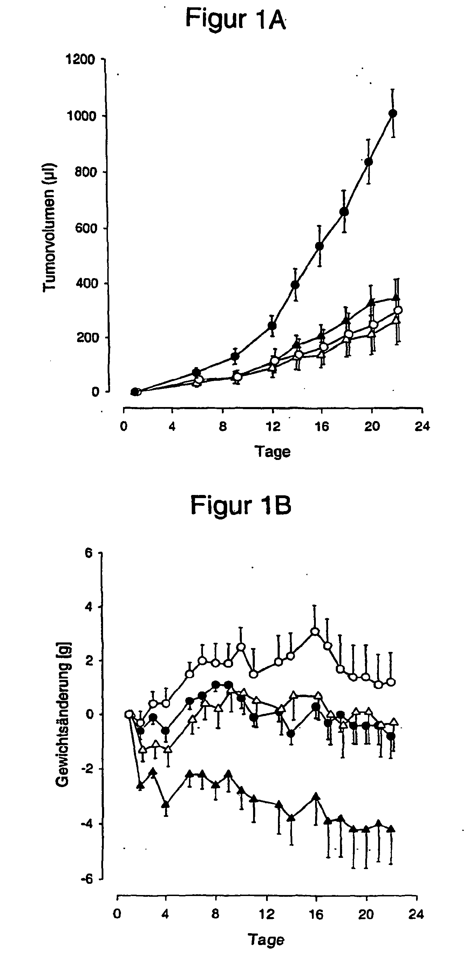

In einem pharmakologisch anerkannten Tiermodell wurde Nacktmäusen (NMRInu/nu; Harlan, Borchen) eine Zellsuspension (5 x 10⁶ Zellen in 300 µl pro Maus) aus menschlichen Brustkrebszellen (MCF-7 Zelllinie) subkutan injiziert. Ober 3 Wochen wurden die Tiere aus der Kontrollgruppe mit physiologischer Kochsalzlösung und die Tiere aus den drei Behandlungsgruppen mit Morphin, Naloxon oder der Kombination aus Morphin und Naloxon behandelt **(Fig. 1** mit genauerer Versuchsbeschreibung). Die wöchentlich steigende Dosis sollte die bekannte Toleranzentwicklung gegenüber Morphin ausgleichen. Die Naloxondosis betrug 1/10 der Morphindosis, weil bei diesem Dosierungsverhältnis bekannterweise die Morphineffekte vollkommen antagonisiert werden. Gegenüber der Kontrollgruppe senkte die Morphinbehandlung das Tumorwachstum innerhalb von 3 Wochen statistisch hochsignifikant auf etwa ein Drittel.(Fig. 1 A) Überraschenderweise zeigte die Kombination aus Morphin und Opioid-Antagonist Naloxon einen mindestens gleich guten bis besseren Effekt. Wegen der vollkommenen Antagonisierung der für Morphin typischen pharmakologischen Wirkungen durch Naloxon kann aus diesen Ergebnissen abgeleitet werden, dass auch andere Wirkmechanismen als die für die Schmerzstillung verantwortlichen im wesentlichen zur Antitumorwirkung von Morphin beitragen. Fig. 1 B zeigt als eine bekannte unerwünschte Wirkung eine Abnahme des Körpergewichtes bei den mit Morphin behandelten Mäusen. In der Kontrollgruppe und in der Gruppe mit Morphin+Naloxon-Behandlung blieb das Körpergewicht unbeeinflusst. In der Naloxongruppe nahm das Körpergewicht leicht zu.

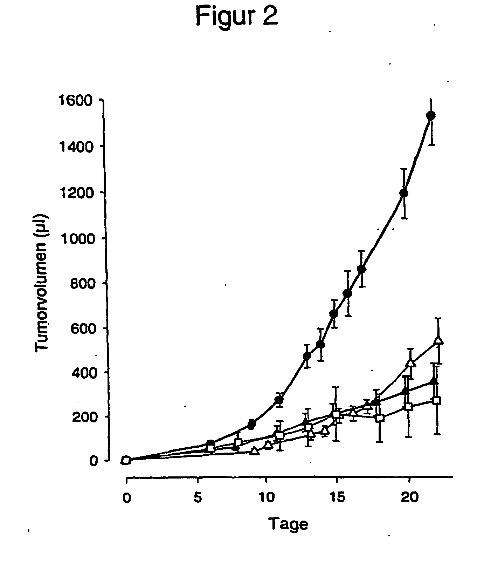

In einem ähnlich konzipierten Tierversuch **(Fig. 2)** wurde zur Verifizierung der überraschenden Ergebnisse Morphin, Morphin-6-glucuronid (ein Morphinmetabolit) und die Kombination aus Morphin+Naltrexon (ein Opioid-Antagonist) gegen eine Kontrolle verglichen. Es bestätigte sich die gute antitumorale Wirkung von Morphin und die ähnlich gute bis bessere Wirkung von Morphin in Kombination mit dem Opioid-Antagonisten Naltrexon. Morphin-6-glucuronid erwies sich als ähnlich wirksam bis etwas weniger wirksam als Morphin. Um die Erfindung bezüglich der therapeutischen Relevanz weiter einordnen zu können sowie die Übertragbarkeit auf ähnliche Wirkstoffe herstellen zu können, wurden systematische Untersuchungen zum Mechanismus der entdeckten vorteilhaften Wirkungen durchgeführt.

Als Modellsubstanzen für die weiteren Untersuchungen wurden DAMGO {(D-Ala2, N-Methyl-Phe4, Gly-ol5) enkephalin}, Naloxon und die Kombination aus beiden Wirkstoffen eingesetzt. Diese Substanzen werden in der pharmakologischen Forschung häufig eingesetzt, um die allgemeinen Wirkprinzipien von Opioiden aufzuklären. Als Opioide bezeichnet man alle Substanzen, deren Wirkung über die Bindung an die Opioid-Rezeptoren im Organismus zustande kommt. Hierzu zählen die zahlreichen Opiumalkaloide aus den Samenkapseln von Papaver somniferum, von denen Morphin die größte medizinische Bedeutung zukommt. Aus den Opiumalkaloiden werden durch partialsynthetische Umwandlung weitere wirksame Substanzen hergestellt. Im Organismus werden über ein körpereigenes schmerzhemmendes System Opioidpeptide, z. B. Endorphine, Enkephaline und Dynorphine gebildet. Schließlich stehen vollsynthetisch hergestellte Wirkstoffe zur Verfügung, die über die Bindung an die Opioid-Rezeptoren pharmakologisch wirksam werden.

Die an die Opioid-Rezeptoren bindenden Substanzen können agonistisch (erregend), antagonistisch (hemmend), oder gemischt agonistisch und antagonistisch wirken. Dieser Unterschied und die Tatsache, dass sich die Opioid-Rezeptoren noch in Subtypen (µ-, κ-, δ-Rezeptoren) unterteilen, die unterschiedliche physiologische Funktionen wahrnehmen, macht deutlich, dass sich die an die Opioid-Rezeptoren bindenden Substanzen in der Wirkqualität und im Ausmaß der Wirkungen unterscheiden können. In den wesentlichen Wirkungen verhalten sich jedoch alle Agonisten und alle Antagonisten untereinander ähnlich. Die für die weiteren Untersuchungen ausgewählte Modellsubstanz DAMGO hat sich als selektiver µ-Agonist in der pharmakologischen Forschung weitgehend etabliert, um die für den klinischen Effekt von Opioiden wichtigste schmerzhemmende Wirkung sowie die unerwünschten Wirkungen gezielt untersuchen zu können. Mit Erkenntnissen, die mit DAMGO gewonnen werden, kann auf Grund der vorliegenden Erfahrung auf andere Opioide mit vorwiegend µ-agonistischer Wirkung geschlossen werden. Naloxon ist der in der pharmakologischen Forschung und in der klinischen Anwendung am weitesten verbreitete Opioid-Rezeptorantagonist, der an allen bekannten Opioid-Rezeptorsubtypen antagonistisch wirkt.

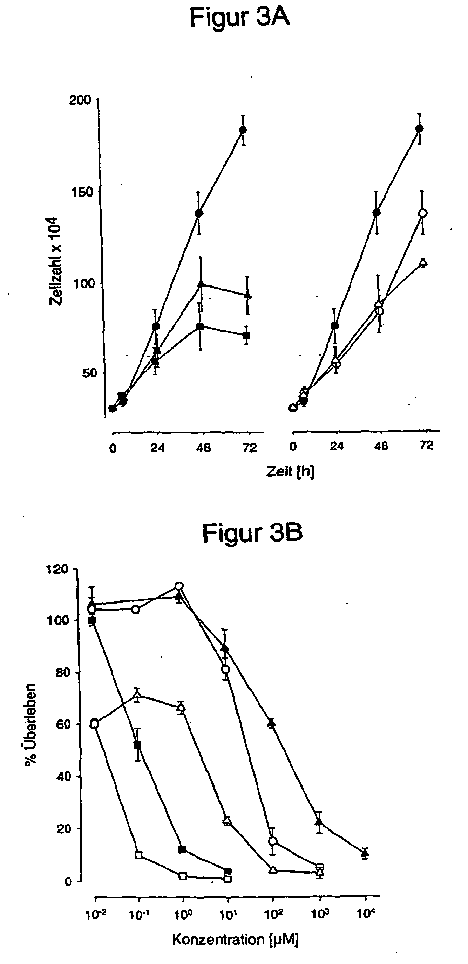

In einem Zellproliferationstest mit menschlichen MCF-7-Brustkrebszellen wurde die Wirkung von DAMGO, Naloxon und der Mischung aus beiden Wirkstoffen gemessen **(Fig. 3 A).** Gegenüber der unbehandelten Kontrolle reduzierte DAMGO konzentrationsabhängig den Zellteilungszyklus erheblich. Bei den mit DAMGO behandelten Kulturen wurde die maximale Zellzahl nach 48 Stunden erreicht. Zwischen 48 und 72 Stunden starb ein Teil der Zellen unter DAMGO-Behandlung ab (Fig. 3 A links). Interessanterweise führte auch die Behandlung mit Naloxon und der Mischung aus Naloxon und DAMGO zu einem wachstumshemmenden Effekt (Fig. 3 A rechts). Folglich wurde der wachstumshemmende Effekt von DAMGO durch Naloxon nicht antagonisiert.

An derselben Zelllinie wurde der cytotoxische Effekt der Wirkstoffe durch Bestimmung der Zellüberlebensraten bestimmt **(Fig. 3 B).** DAMGO und interessanterweise auch wiederum Naloxon zeigten einen konzentrationsabhängigen positiven Effekt auf die Überlebensrate. Wenn zu 100 µMol DAMGO steigende Konzentrationen Naloxon zugesetzt wurden, verschob sich die Überlebenskurve deutlich nach links. Dies zeigt einerseits, dass die Mischung aus beiden Wirkstoffen wesentlich besser wirkt als die Substanzen alleine und, dass andererseits zwischen DAMGO und Naloxon kein gegenseitiger Antagonismus auftritt. TNFα diente als Positivkontrolle. Durch DAMGO-Zugabe konnte die Wirkung von TNFα nochmals erheblich gesteigert werden.

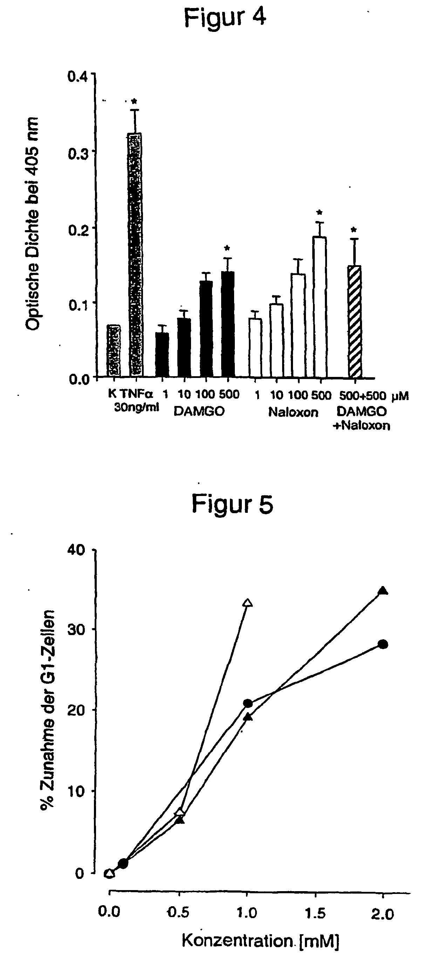

Zur näheren Charakterisierung des apototischen Zelltodes wurde mit einem kommerziell verfügbaren in der Wissenschaft üblichen Apotosemesskit die Rate der DNA Spaltung gemessen **(Fig. 4).** TNFα, von dem bekannt ist dass es die Apoptose in MCF-7 Zellen einleitet, wurde als Positivkontrolle verwendet. Im Vergleich zur unbehandelten Kontrolle nahm mit 500 µMol DAMGO, 500 µMol Naloxon und 500 µMol DAMGO+500 µMol Naloxon die Rate der DNA Spaltung signifikant zu.

Da es sich um ein Peptid handelt, wird der selektive µ-Agonist DAMGO zur Zeit klinisch nicht eingesetzt. Um die Übertragbarkeit der damit erhaltenen Ergebnisse auf den klinisch am häufigsten eingesetzten Opioid-Agonisten Morphin mit relativ selektiver Wirkung als µ-Agonist, aber auch mit schwacher Bindung an δ- und κ-Opioid-Rezeptoren, zu gewährleisten, wurden Zellzyklusanalysen an MCF-7 Zellen nach.Behandlung mit Morphin, Naloxon oder Morphin+Naloxon durchgeführt **(Fig. 5).** Gemessen wurde der Anteil von Zellen in der G1-Phase, die innerhalb der Interphase des Zellzyklus vor der S-Phase (Synthese der DNA) steht. Die konzentrationsabhängige Zunahme von Zellen in der G1-Phase zeigt, dass Morphin das Fortschreiten des Zellzyklus von der G1- in die S-Phase hemmt. Überraschenderweise wurde auch bei Naloxon ein ähnlicher Zellzyklusblock gefunden. Die Kombination aus beiden Wirkstoffen war stärker wirksam als jeder Wirkstoff alleine, was einen additiven Effekt der Kombination anzeigt.

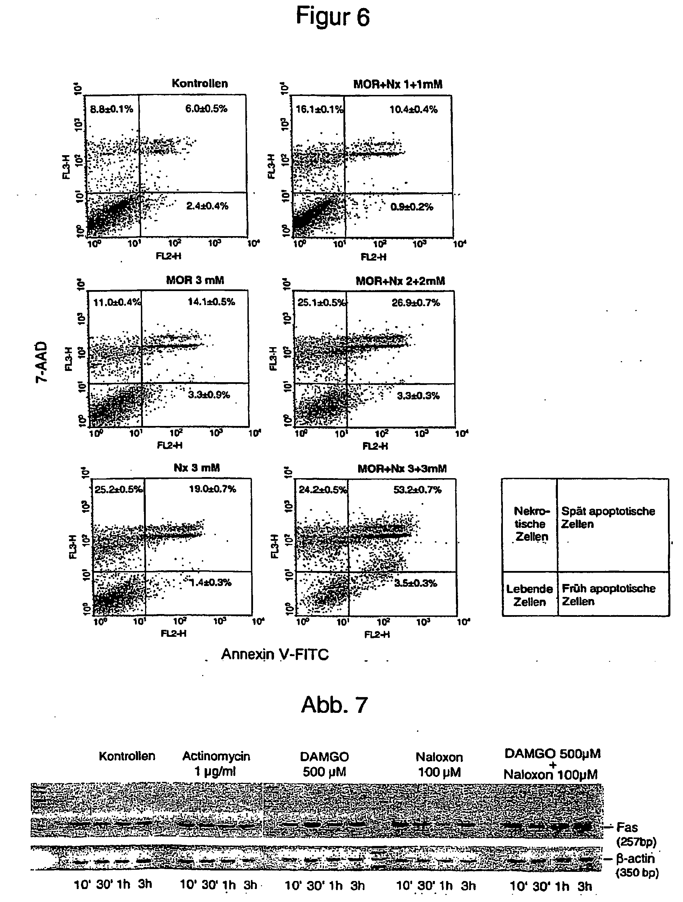

Zur weiteren Beschreibung der Apoptose durch Morphin, Naloxon oder der Mischung aus beiden Wirkstoffen wurde unter dem Einfluss der Wirkstoffe die Bindung von Annexin V an Phosphatidylserin der Plasmamembran und die 7-Amino-actinomycin D (7-AAD)-DNA-Bindung mittels der Flow-Cytometrie gemessen **(Fig. 6).** Bei Konzentrationen von 3 mMol zeigten Morphin und Naloxon eine deutliche Zunahme von Zellen in der späten Apoptose. Bei 2 mMol war der Efekt schwächer ausgeprägt und bei 1 mMol (nicht abgebildet) nicht mehr feststellbar. Die Wirkstoffmischung war wiederum stärker wirksam als die Einzelwirkstoffe. Eine gegenseitige Antagonisierung dieser Wirkung zwischen Morphin und Naloxon trat nicht ein.

Aus den dargestellten und weiteren erfindungsgemäß durchgeführten Untersuchungen kann das Fazit gezogen werden, dass im Modellversuch mit menschlichen MCF-7 Brustkrebszellen Wirkstoffe aus der Gruppe der µ-Opioid-Rezeptoragonisten, insbesondere das klinisch bedeutsame Morphin, einen Zellzyklusblock und eine Zunahme der Zelltodesrate hervorrufen. Entsprechend wird in dem anerkannten pharmakologischen Modell mit dieser Zelllinie das Tumorwachstum bei Nacktmäusen gehemmt. Die Konzentrationen von Morphin im Plasma dieser Tiere (nicht abgebildet) entsprachen den Plasmaspiegeln bei Patienten unter chronischer oraler Morphingabe zur Schmerzbehandlung. Eine wesentliche gegenseitige Antagonisierung der geprüften Wirkungen trat nicht ein. Die Kombination aus Agonist und Antagonist war mindestens gleich wirksam, oder wirksamer als die Einzelsubstanzen. Insgesamt verursachen die Substanzen einen teilweise über den µ-Rezeptor vermittelten Mechanismus einschließlich einer Phosphorylierung und Stabilisierung von p53 und einer Hochregulierung von p53 abhängigen Proteinen einschließlich p21, Bax und des "Todesrezeptors" CD95/Fas **(Fig. 7)** (im einzelnen nur teilweise dargestellt). Das Fehlen eines gegenseitigen Antagonismus zwischen µ-Agonist und Antagonist bei einem wesentlichen Teil der gezeigten Wirkungen macht deutlich, dass ein Teil der Effekte unabhängig von den bekannten µ-Rezeptorwirkungen vermittelt wird.

Die erfindungsgemäß durchgeführten Untersuchungen zeigen, dass µ-Opioid-Rezeptoragonisten als Wirkstoffe zur Krebsbehandlung geeignet sind. Dies gilt für selektive sowie für teilselektive µ-Agonisten. Durch Kombination dieser Wirkstoffe mit Opioid-Rezeptorantagonisten können die für die Agonisten gefundenen Wirkungen verstärkt beziehungsweise unter Reduzierung der für die Opioid-Rezeptoragonisten typischen Nebenwirkungen zumindest voll erhalten werden.

Bei der kombinierten Anwendung von µ-Opioid-Rezeptoragonisten und Opioid-Rezeptorantagonisten können die Dosierungsverhältnisse den individuellen therapeutischen Bedürfnissen angepasst werden. Schmerzfreie Patienten werden mit einer krebswirksamen Dosis des Agonisten zusammen mit einem Anteil des Antagonisten so kombiniert, dass die typischen Opioidwirkungen vollkommen aufgehoben werden. Dadurch können die zum Teil erheblichen Nebenwirkungen dieser Substanzklasse weitgehend verhindert werden. Wegen des reduzierten oder vollkommen ausgeschalteten Abhängigkeitspotenzials können somit Opioid-Rezeptoragonisten, insbesondere das klinisch bedeutsame Morphin, ohne die sonst üblichen Restriktionen klinisch eingesetzt werden. Je nach Schwere der Schmerzen kann der relative Anteil an Opioid-Rezeptoragonisten so weit erhöht werden, dass neben einer ausreichenden Dosis für die Krebstherapie auch eine ausreichende schmerzlindernde Wirkung durch einen relativen Dosisüberschuss des Agonisten erreicht wird. Bei der kombinierten Anwendung kann die Dosis des Agonisten dem klinischen Bild entsprechend höher gewählt werden, weil durch vollkommene oder teilweise Ausschaltung der µ-Rezeptorwirkung durch den Antagonisten die typischen Nebenwirkungen minimiert werden. Da bezüglich der Wirksamkeit gegen Krebs praktisch keine gegenseitige Antagonisierung der Effekte durch die Kombinationspartner zustande kommt, kann durch die im Vergleich zur Monosubstanz höhere Dosis des Agonisten ein maximaler erwünschter Effekt bei deutlich reduzierten Nebenwirkungen erzielt werden.

Bei Krebspatienten mit Schmerzen können zu der Mischung aus Opioid-Rezeptoragonisten und -antagonisten andere Schmerzmittel wie Paracetamol, Acetylsalicylsäure, nichtsteroidale Entzündungshemmer, selektive COX 2-Hemmer, etc. zugegeben werden. Diese Substanzen zeigen in vielen Fällen wegen des andersartigen Wirkungsmechanismus eine zusätzliche Schmerzstillung im Vergleich zur alleinigen Anwendung von Opioid-Rezeptoragonisten. Bei Schmerzlinderung über diese zusätzlichen Kombinationspartner kann ein hoher Anteil des Antagonisten in Relation zum Agonisten in das Arzneimittel mit der Folge eingebracht werden, dass eine optimale Wirkung gegen Krebs bei minimalen Nebenwirkungen und guter Schmerzlinderung erzielt wird.

Die einfachste Art der gleichzeitigen Anwendung von µ-Opioid-Rezeptoragonisten und Opioid-Rezeptorantagonisten, eventuell in Verbindung mit anderen schmerzlindernden Mitteln ist die Zubereitung in einer Arzneiform. Durch unterschiedliche Dosierungsverhältnisse zwischen Agonist und Antagonist können Fertigarzneiformen für die obengenannten individuellen therapeutischen Bedürfnisse hergestellt werden. Agonist, Antagonist und eventuell das zusätzliche Schmerzmittel können auch in getrennten Arzneiformen zur Verfügung gestellt werden. Damit wäre im Einzelfall eine noch weiter auf den individuellen Patienten hin optimierte Behandlung durch individuelle Wahl der Dosis und des Dosierungsintervalls möglich.

Die Arzneimittel können nach allen medizinisch etablierten Applikationsarten verabreicht werden. In Frage kommen z.B. Formen, die intravenös, intraarteriell (z. B. in die tumorzuführende Arterie) intramuskulär, subkutan, intraperitoneal, oral, buccal, rektal, topisch, transdermal,epidural, intrathekal, oder lokal in den Tumor verabreicht werden. Als mögliche Zubereitungen kommen z. B. Lösungen, Suspensionen, Emulsionen, Tabletten, Kapseln, Trinkzubereitungen, Suppositorien, halbfeste Zubereitungen, transdermale therapeutische Systeme und Inhalationsformulierungen in Frage. Die Herstellung dieser Zubereitungen erfolgt mit den in der pharmazeutischen Technologie üblichen Hilfsstoffen. Zur optimalen Zubereitung und Applikation können die umfangreichen Erkenntnisse zum biopharmazeutischen und pharmakokinetischen Verhalten sowie zur üblichen medizinischen Anwendung dieser gut bekannten Einzelstoffe herangezogen werden.

Anhand der folgenden Figuren wird die vorliegende Erfindung näher erläutert. Dabei zeigen:
- **Fig. 1 A**: MCF-7 Tumorwachstum in NMRI nu/nu Nacktmäusen (Harlan, Borchen) nach Behandlung mit Morphin (▲; n = 15), Naloxon (○; n = 8), Morphin + Naloxon (Δ; n = 11) oder Vehikel (●; n = 13). 5 x 10⁶ Zellen wurden pro Maus subkutan injiziert. Die Morphin- und Naloxondosen betrugen 10, 20 und 30 mg/kg täglich in der 1., 2. und 3. Woche. Für die Wirkstoffkombination wurden tägliche Morphindosen von 10, 20 und 30 mg/kg und Naloxondosen von 1, 2 und 3 mg/kg verwendet. Einen Tag vor, sowie 7 und 14 Tage nach der Tumorzellinjektion wurden die Tiere mit 10 mg/kg β-Estradiolvalerat (i.m.) behandelt. Diese Substanz fördert das Tumorwachsturn und wird deshalb in diesem Tiermodell standard-gemäß eingesetzt. Tumorvolumen = (langer Durchmesser x kurzer Durchmesser²) / 2
- **Fig. 1 B**: Körpergewichtsentwicklung während der Behandlung. Behandlung wie in 1 A.
- **Fig. 2**: MCF-7 Tumorwachstum in NMRI nu/nu Nacktmäusen nach Behandlung mit Vehikel (Kontrolle; n = 15), Morphin (n = 10), Morphin-6-glucuronid (n = 5) oder Morphin + Naltrexon (n = 5). Am Tag 0 wurden 4 x 10⁶ MCF-7 Zellen subkutan injiziert. Folgende tägliche Dosen wurden verabreicht: Morphin: 10, 15 oder 20 mg/kg in der 1., 2. und 3. Woche; Morphin-6-glucuronid: 10 mg/kg; Morphin + Naltrexon: 10 + 10, 15 + 15 oder 20 + 20 mg/kg in der 1., 2. oder 3 Woche
- **Fig. 3 A**: MCF-7 Zellproliferation in vitro nach Behandlung mit 100 µMol DAMGO (▲), 500 µMol DAMGO (■) (links), 100 µMol Naloxon (○) oder der Mischung aus 500 µMol DAMGO + 100 µMol Naloxon (Δ) (rechts) im Vergleich zu unbehandelten Kontrollzellen (●). Mittelwerte ± SEM aus 4 - 6 Wiederholexperimenten
- **Fig. 3 B**: Überlebensrate vom MCF-7 Zellen nach Behandlung mit DAMGO (▲), Naloxon (○), 100 µMol DAMGO + Naloxon (Δ), TNFα (■) und 100 µMol DAMGO + TNFα (□). Die Überlebensrate wurde auf die Zahl der unbehandelten Kontrollzellen mit 100 % normiert. Mittelwerte ± SEM aus 4 Wiederholexperimenten
- **Fig. 4**: DNA Spaltungsrate nach Inkubation von MCF-7 Zellen für 24 Stunden mit den in der Abszisse angegebenen Wirkstoffdosen. Mittelwerte ± SEM aus 6 unabhängigen Experimenten. Messung der DNA-Spaltung über die Bestimmung der Mono- und Oligonucleosomen mit einem Apoptose-Detektions-Kit, der Antihistone und anti-DNA Antikörper enthält.
- **Fig. 5**: Zellzyklusanalyse mit FACS-Analyse von MCF-7 Zellen 24 Stunden nach Behandlung mit Morphin (▲) oder Naloxon (●) in den Konzentrationen 0,5, 1,0 und 2,0 mMol sowie nach Behandlung mit Morphin + Naloxon (Δ) in den Konzentrationen 0,5 mMol Morphin + 0,1 mMol Naloxon und 1 mMol Morphin + 1 mMol Naloxon. Mittelwerte aus 3 unabhängigen Experimenten.
- **Fig. 6**: Flow Cytometrie Analyse der Annexin V und 7-AAD Bindung zur Bestimmung der Apoptoserate nach 24 Stunden Behandlung mit Morphin (MOR), Naloxon (Nx) oder der Kombination aus Morphin und Naloxon in den angegebenen Konzentrationen.
- **Fig. 7**: Expression der CD95/Fas mRNA in MCF-7 Zellen nach Behandlung mit Actinomycin D (Positivkontrolle), DAMGO, Naloxon oder DAMGO + Naloxon in den angegebenen Konzentrationen und Behandlungszeiten. Der Fas mRNA Gehalt wurde durch rt-PCR unter Verwendung von 1 µg Gesamt-RNA bestimmt. Repräsentative Ergebnisse aus 3 unabhängigen Experimenten.

## Patentansprüche

1. Verwendung von Wirkstoffen mit µ-Opioid-Rezeptor agonistischer Wirkung ausgewählt unter Morphin, Morphin-6-glucuronid, Methadon, Pethidin, Fentanyl, Tramadol, Pentazocin, Buprenorphin, Tilidin, Levomethadon, Piritramid, Levacetylmethadol, Codein, Oxycodon, Hydromorphon, Dihydrocodein, Diamorphin, Sufentanil, Alfentanil, Remifentanil, Nalbuphin, Butorphanol, deren Salzen und Glucuroniden sowie Mischungen dieser Substanzen und Opioid-Rezeptor antagonistischer Wirkung ausgewählt unter Naloxon und Naltrexon und deren Salzen sowie Mischungen dieser Substanzen als Kombinationsarzneimittel zur Krebsbehandlung.

2. Verwendung gemäß Anspruch 1, **dadurch gekennzeichnet, dass** es sich bei dem Krebs um Brustkrebs handelt.

3. Verwendung nach Anspruch 1 oder 2, **dadurch gekennzeichnet, dass** die µ-Opioid-Rezeptoragonisten und die Opioid-Rezeptorantagonisten in einer Arzneiform kombiniert werden oder einzeln zubereitet in einer Kombipackung oder einzeln zubereitet in getrennten Arzneipackungen für die Therapie zur Verfügung gestellt werden.

4. Verwendung nach einem der Ansprüche 1 bis 3, **dadurch gekennzeichnet, dass** den µ-Opioid-Rezeptoragonisten und Opioid-Rezeptorantagonisten weitere schmerzlindernde oder entzündungshemmende Wirkstoffe, ausgewählt unter Paracetamol, Acetylsalicylsäure, nichtsteroidalen Antirheumatika, selektiven COX 2-Hemmern und/oder TNFα-Hemmern hinzu gegeben werden.

5. Verwendung nach einem der Ansprüche 1 bis 4, **dadurch gekennzeichnet, dass** aus den Wirkstoffen zusammen mit pharmazeutisch und pharmakologisch verträglichen Hilfsstoffen fertige Arzneimittel wie Lösungen zur Injektion oder Infusion, Suspensionen, Emulsionen, Tabletten, Kapseln, Trinkzubereitungen, Kauformulierungen, Suppositorien, halbfeste Zubereitungen, transdermale therapeutische Formulierungen oder Inhalationsformulierungen hergestellt werden.

6. Verwendung nach einem der Ansprüche 1 bis 4, **dadurch gekennzeichnet, dass** aus den Wirkstoffen zusammen mit pharmazeutisch und pharmakologisch verträglichen Hilfsstoffen transdermale therapeutische Systeme mit langzeitig kontinuierlicher oder intermittierender Freisetzungscharakteristik, insbesondere mit den Wirkstoffen Fentanyl und Buprenorphin, hergestellt werden.

7. Verwendung nach einem der Ansprüche 1 bis 6, **dadurch gekennzeichnet, dass** die Arzneimittel intravenös, intraarteriell, intramuskulär, subkutan, intraperitoneal, oral, buccal, nasal, rektal, topisch, transdermal, epidural, intrathekal oder lokal in den Tumor appliziert werden können.

## Claims

1. Use of active ingredients having a µ-opioid receptor agonist action chosen among morphine, morphine-6-glucuronide, methadone, pethidine, fentanyl, tramadol, pentazocine, buprenorphine, tilidine, levomethadone, piritramide, levacetylmethadole, codeine, oxycodone, hydromorphone, dihydrocodeine, diamorphine, sufentanil, alfentanil, remifentanil, nalbuphine, butorphanol, their salts and glucuronides as well as mixtures of these substances and opioid receptor antagonist action chosen among naloxone or naltrexone and their salts as well as mixtures of these substances, as combination drugs for the treatment of cancer.

2. Use according to claim 1 **characterized in that** the cancer is breast cancer.

3. Use according to Claim 1 or 2, **characterized in that** the µ-opioid receptor agonists and opioid receptor antagonists are combined into a single form of administration or prepared individually and supplied for therapy in a combination package or prepared individually and supplied for therapy in separate drug packages.

4. Use according to any one of Claims 1 to 3, **characterized in that** the µ-opioid receptor agonists and opioid receptor antagonists are supplemented with other pain-relieving or anti-inflammatory active ingredients, chosen among paracetamol, acetylsalicylic acid, non-steroidal anti-inflammatory drugs, selective COX-2 inhibitors, and/or TNFα inhibitors.

5. Use according to any one of Claims 1 to 4, **characterized in that** the active ingredients are used together with pharmaceutically and pharmacologically compatible excipients to manufacture finished pharmaceutical products, such as solutions for injection or infusion, suspensions, emulsions, tablets, capsules, potable preparations, chewable formulations, suppositories, semisolid preparations, transdermal therapeutic formulations or formulations for inhalation.

6. Use according to any one of Claims 1 to 4, **characterized in that** the active ingredients are used together with pharmaceutically and pharmacologically compatible excipients to manufacture transdermal therapeutic systems with long-term continuous or intermittent release characteristics, in particular those containing the active ingredients, fentanyl and buprenorphine.

7. Use according to any one of Claims 1 to 6, **characterized in that** the drugs can be applied by intravenous, intraarterial, intramuscular, subcutaneous, intraperitoneal, oral, buccal, nasal, rectal, topical, transdermal, epidural, intrathecal application or locally into the tumor.

## Revendications

1. Utilisation de principes actifs possédant une activité agoniste vis-à-vis du récepteur opioïde mu, choisis parmi la morphine, la morphine-6-glucuronide, la méthadone, la péthidine, le fentanyl, le tramadol, la pentazocine, la buprénorphine, la tilidine, la lévométhadone, le piritramide, le lévacétylméthadol, la codéine, l'oxycodon, l'hydromorphone, la dihydrocodéine, la diamorphine, le sufentanil, l'alfentanil, le remifentanil, la nalbuphine, le butorphanol, leurs sels et leurs glucuronides, et des mélanges de ces substances, et une activité antagoniste vis-à-vis des récepteurs opioïdes, choisis parmi la naloxone et la naltrexone et leurs sels, et des mélanges de ces substances, à titre de médicament de combinaison pour le traitement du cancer.

2. Utilisation selon la revendication 1, **caractérisée en ce qu'**il s'agit, en ce qui concerne le cancer, du cancer du sein.

3. Utilisation selon la revendication 1 ou 2, **caractérisée en ce que** les agonistes du récepteur opioïde mu et les antagonistes des récepteurs opioïdes sont combinés en une forme galénique ou bien sont mis à disposition en étant préparés individuellement dans un conditionnement de type combiné ou en étant préparés individuellement dans des conditionnements galéniques séparés pour la thérapie.

4. Utilisation selon l'une quelconque des revendications 1 à 3, **caractérisée en ce que** d'autres principes actifs analgésiques ou anti-inflammatoires choisis parmi le paracétamol, l'acide acétylsalicylique, des antirhumatoïdes non stéroïdiens, des inhibiteurs sélectifs de Cox-2 et/ou des inhibiteurs du TNF-α sont ajoutés aux agonistes du récepteur opioïde mu et aux antagonistes des récepteurs opioïdes.

5. Utilisation selon l'une quelconque des revendications 1 à 4, **caractérisée en ce que**, à partir des principes actifs, conjointement avec des adjuvants pharmaceutiquement et pharmacologiquement acceptables, on prépare des médicaments prêts à l'emploi tels que des solutions pour injection ou pour perfusion, des suspensions, des émulsions, des comprimés, des capsules, des préparations buvables, des formulations à mâcher, des suppositoires, des préparations semi-solides, des formulations thérapeutiques transdermiques ou des formulations pour inhalation.

6. Utilisation selon l'une quelconque des revendications 1 à 4, **caractérisée en ce que**, à partir des principes actifs, conjointement avec des adjuvants pharmaceutiquement et pharmacologiquement acceptables, on prépare des systèmes thérapeutiques transdermiques possédant une caractéristique de libération continue ou intermittente à long terme, en particulier avec les principes actifs fentanyl et buprénorphine.

7. Utilisation selon l'une quelconque des revendications 1 à 6, **caractérisée en ce qu'**on peut appliquer les médicaments par voie intraveineuse, par voie intraartérielle, par voie intramusculaire, par voie sous-cutanée, par voie intrapéritonéale, par voie orale, par voie buccale, par voie nasale, par voie rectale, par voie topique, par voie transdermique, par voie épidurale, par voie intrathécale ou par voie locale dans la tumeur.
